# EUROPEAN PATENT APPLICATION

(11) **EP 4 556 463 A1**
(43) Date of publication of application: **21.05.2025**
(21) Application number: 23860611.5
(22) Date of filing: 08.06.2023
(51) Int. Cl.: C07C 309/71, C07C 303/30, A61K 8/46, A61Q 17/04

(54) **ORGANIC SALT OF TEREPHTHALYLIDENE DICAMPHOR SULFONIC ACID FOR USE IN UV-BLOCKING COSMETIC COMPOSITIONS, AND PREPARATION METHOD THEREFOR**

(30) Priority: 31.08.2022 KR 20220110245; 07.06.2023 KR 20230073128
(71) Applicant: Shinsung Materials Co., Ltd., Chungcheongbuk-do 27850 (KR)
(72) Inventor: KIM, Kwang Shik, Jincheon-gun, Chungcheongbuk-do 27850 (KR); SHIN, Sang Kyu, Jincheon-gun, Chungcheongbuk-do 27850 (KR)
(74) Representative: BCKIP Part mbB
(86) International application number: PCT/KR2023/007815
(87) International publication number: WO 2024/048925

(57) **Abstract**

The present invention relates to an organic salt of terephthalylidene dicamphor sulfonic acid, which is useful as a UV-blocking cosmetic composition, and its manufacturing method.

The present invention provides an organic salt of terephthalylidene dicamphor sulfonic acid, which is obtained as a neutralized product having a pH of 5.0 to 9.0 by combining an organic amine compound with a strongly acidic pH 1 aqueous solution of terephthalylidene dicamphor sulfonic acid, in an aqueous solution phase or in a powder phase, and thus the present invention allows manufacturing cosmetics without additionally using a neutralizing agent, providing convenience and safety, and has excellent long-term storage stability, enabling consistent prescription, especially in cosmetic formulations.

## Description

### Technical Field

The present invention relates to an organic salt of terephthalylidene dicamphor sulfonic acid, which is useful for UV-blocking cosmetic compositions, and manufacturing method thereof. More specifically, the present invention provides an organic salt of terephthalylidene dicamphor sulfonic acid, which is obtained as a neutralized product having a pH of 5.0 to 9.0 by combining an organic amine compound with a strongly acidic pH 1 aqueous solution of terephthalylidene dicamphor sulfonic acid, in an aqueous solution phase or in a powder phase, and thus the present invention allows manufacturing cosmetics without additionally using a neutralizing agent, providing convenience and safety, and has excellent long-term storage stability, enabling consistent prescription of cosmetic formulations.

### Background Art

Due to the destruction of the ozone layer caused by climate change, UV rays directly hit human skin, causing spots and skin aging. Therefore, UV blockers are indispensable for cosmetics and are widely used in cosmetics.

Particularly, the wavelength range of 280-320 nm, known as UVB, causes erythema (redness) of the human skin, while the wavelength range of 320-400 nm, known as UVA, causes browning of the skin, leading to skin damage and contributing to the development of skin cancer.

Moreover, many countries around the world are strengthening regulations regarding skin safety and irritation for sunscreens that are applied to the skin.

When light in the UV range enters a UV blocker, it absorbs the light energy of the UV rays and changes it into heat energy, in other words, the absorbed light ends its life as light and changes into heat. The UV light is absorbed by UV blockers and blocked from reaching the skin, and thus the skin is protected.

Currently, terephthalylidene dicamphor sulfonic acid (TDSA, referred to as "Ecamsule" in the US Pharmacopoeia) is a UV blocker approved by the US FDA for its blocking performance in the wavelength range of 320 to 400 nm corresponding to UV A.

As the need for UV blocking increases day by day, terephthalylidene dicamphor sulfonic acid is expanding its application to various fields such as hair rinses and medicine as well as cosmetics due to the expansion of the market.

Patent Documents 1 and 2 disclose methods of synthesizing terephthalylidene dicamphor sulfonic acid, but it is difficult to remove charged organic impurities, phosphorus chloride, sulfate ions, chloride ions, heavy metals, sodium, and the like generated during the synthesis process of terephthalylidene dicamphor sulfonic acid, using only the known synthesis technologies.

Therefore, Patent Document 3 discloses a method of purifying terephthalylidene dicamphor sulfonic acid by using a mixed resin containing cations and anions and thus removing a cationic material and an anionic material generated during the synthesis and precipitation process, thereby improving absorbance at maximum absorption wavelength in UV spectroscopic analysis.

However, since terephthalylidene dicamphor sulfonic acid itself has a strong acidity of pH 1 or lower, it necessarily requires the use of neutralizing agents when used in cosmetic compositions, which creates inconvenience and safety concerns requiring user attention. In particular, even with the use of neutralizing agents, there are problems such as pH adjustment issues and inconsistent formulation in cosmetic applications.

In addition, since terephthalylidene dicamphor sulfonic acid itself is strongly acidic and has a pH of 1 or lower, there is a problem that its purity deteriorates over time because it is present under acidic conditions for a long time.

Accordingly, as a result of making efforts to solve the above-described problems regarding terephthalylidene dicamphor sulfonic acid used as a UV blocker in cosmetics, the present inventors added an organic amine used in cosmetics to an aqueous solution of terephthalylidene dicamphor sulfonic acid having a strong acidity of pH 1 to adjust the pH of the solution to a range of 5.0 to 9.0 and provided an organic salt of terephthalylidene dicamphor sulfonic acid in the form of an aqueous solution or powder, enabling the manufacture of cosmetics without a separate neutralizing agent, making it convenient and safe to formulate cosmetics with a consistent prescription, and at the same time ensuring long-term storage stability, thereby completing the present invention.
(Patent Document 1) US Patent Publication No. 4,585,597 (published on April 29, 1986)
(Patent Document 2) CN Patent Publication No. 106831503 (published on June 13, 2017)
(Patent Document 3) KR Patent Publication No. 10-1937332 (published on January 11, 2019)

### [Disclosure]

### Technical Problem

An object of the present invention is to provide an organic salt of terephthalylidene dicamphor sulfonic acid that meet convenience and safety requirements in cosmetic manufacturing by providing a neutralized product formed through the stoichiometric combination of strongly acidic terephthalylidene dicamphor sulfonic acid with organic amine compounds.

Another object of the present invention is to provide a manufacturing method for obtaining the organic salt of terephthalylidene dicamphor sulfonic acid in the form of an aqueous solution or powder.

A further object of the present invention is to provide a UV-blocking cosmetic composition containing the aforementioned organic salts of terephthalylidene dicamphor sulfonic acid.

### Technical Solution

To achieve the above-described objects, the present invention provides an organic salt of terephthalylidene dicamphor sulfonic acid, obtained as a neutralized product of pH 5.0 to 9.0 by stoichiometrically combining an organic amine compound to both terminals of terephthalylidene dicamphor sulfonic acid, useful in UV-blocking cosmetic compositions, and represented by Chemical Formula 1 below:

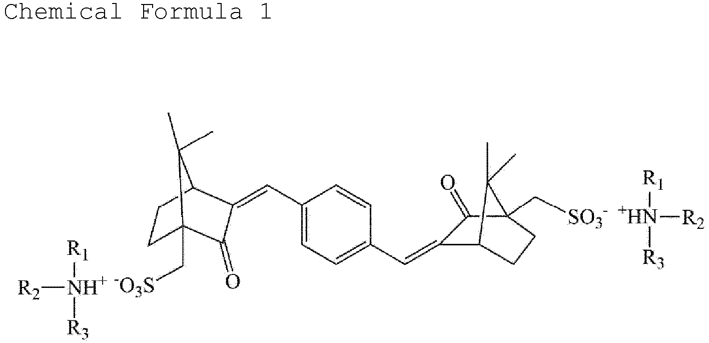

wherein, R₁, R₂, and R₃ are the same or different from each other and independently selected from one or more substituents consisting of hydrogen, hydroxy, amino, carboxy, C₁-C₁₀ alkyl, C₁-C₁₀ hydroxyalkyl, C₁-C₁₀ heteroalkyl, heterocycloalkyl having 20 or fewer carbon atoms, aryl having 20 or fewer carbon atoms, C₁-C₁₀ alkyl containing heterocycloalkyl having 20 or fewer carbon atoms, and C₁-C₁₀ alkyl containing aryl having 20 or fewer carbon atoms.

As the organic amine compound, any one selected from the group consisting of tromethamine, triethanolamine, and 2-amino-2-methyl-1-propanol (AMP) may be preferably used.

As another preferable type of the organic amine compound, any one alkaline amino acid selected from the group consisting of arginine, lysine, histidine, citrulline, and an amino acid derivative in which a carboxyl group of an amino acid is substituted and only a primary amine group remains may be used.

In addition, another preferred type of organic amine compound, any one polyamine selected from the group consisting of polyethyleneimine, spermidine, spermine, cadaverine, and putrescine may be used.

The organic salt of terephthalylidene dicamphor sulfonic acid may be provided in the form of an aqueous solution or powder, thereby providing convenience and safety in the manufacture of cosmetics.

The present invention provides a preparation method of an organic salt of terephthalylidene dicamphor sulfonic acid.

Specifically, the present invention provides a method for preparing an organic salt of terephthalylidene dicamphor sulfonic acid, which is obtained as a clear aqueous solution by dissolving an organic amine compound in a purified terephthalylidene dicamphor sulfonic acid aqueous solution, adjusting the pH of the solution, and then filtering.

In the method of the present invention, a process of adding the above obtained clear aqueous solution dropwise to an organic solvent to precipitate crystals, and drying the crystals in vacuum to obtain in a powder form may further be performed.

In addition, in method of the present invention, a process of freeze-drying the above obtained clear aqueous solution to obtain in a powder form may further be performed.

At this time, when the organic salt of terephthalylidene dicamphor sulfonic acid is an aqueous solution, one or more preservatives selected from the group consisting of 1,2-alkanediol, ethylhexyl glycerin, hydroxyacetophenone, glycerin esters, parabens, and sodium benzoate may further be included to maintain formulation.

### Advantageous Effects

According to the present invention, an organic salt of terephthalylidene dicamphor sulfonic acid obtained as a neutralized product of strongly acidic terephthalylidene dicamphor sulfonic acid and an organic amine compound can be provided, allowing to manufacture cosmetics without using a separate neutralizing agent, providing convenience and safety and ensuring long-term storage stability, thereby enabling consistent formulations in cosmetic applications.

According to the preparation method of the present invention, an organic salt of terephthalylidene dicamphor sulfonic acid can be provided in the dosage form of an aqueous solution or powder.

### Description of Drawings

FIG. 1 shows infrared spectrum results for terephthalylidene dicamphorsulfonic acid·2 tromethamine of the present invention.
FIG. 2 shows a photograph of an aqueous solution of terephthalylidene dicamphorsulfonic acid·2 tromethamine of the present invention.
FIG. 3 shows a photograph of the crystals of terephthalylidene dicamphorsulfonic acid·2 tromethamine of the present invention.
FIG. 4 shows a photograph of the powder of terephthalylidene dicamphorsulfonic acid·2 tromethamine of the present invention.

### Modes of the Invention

The present invention provides an organic salt of terephthalylidene dicamphor sulfonic acid obtained as a neutralized product of pH 5.0 to 9.0 by stoichiometrically combining an organic amine compound to both terminals of terephthalylidene dicamphor sulfonic acid and represented by Chemical Formula 1 below:

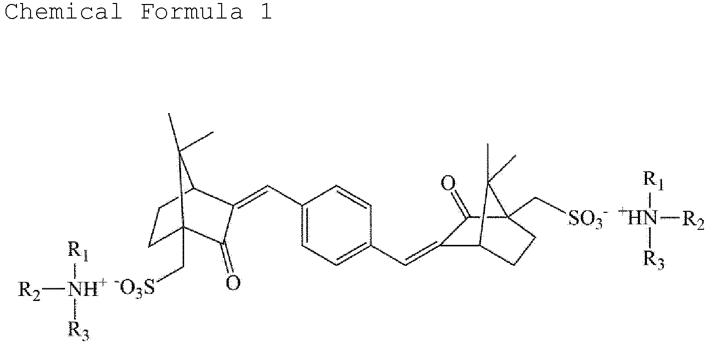

wherein, R₁, R₂, and R₃ are the same or different from each other and independently selected from one or more substituents consisting of hydrogen, hydroxy, amino, carboxy, C₁-C₁₀ alkyl, C₁-C₁₀ hydroxyalkyl, C₁-C₁₀ heteroalkyl, heterocycloalkyl having 20 or fewer carbon atoms, aryl having 20 or fewer carbon atoms, C₁-C₁₀ alkyl containing heterocycloalkyl having 20 or fewer carbon atoms, and C₁-C₁₀ alkyl containing aryl having 20 or fewer carbon atoms.

The terephthalylidene dicamphor sulfonic acid is used as a cosmetic raw material for UV-blocking due to its ability to block UV rays in the wavelength range of 320 to 400 nm known as UV A. However, in order to resolve the problem of strong acidity of terephthalylidene dicamphor sulfonic acid, it is provided in the form of a salt thereof, thereby ensuring safety during use, providing convenience in work, and providing a cosmetic composition having stable UV blocking ability.

The organic amine compounds used to form the above salt can be organic amine compounds used in cosmetic formulations, alkaline amino acids, biologically derived amines, organic amine compounds used as food additives, and organic amine compounds used in pharmaceuticals.

In the organic salt of terephthalylidene dicamphor sulfonic acid of the present invention, the salt used is an organic amine compound designated for use in a pharmaceutical raw material, a food additive raw material, and a cosmetic raw material, and thus the stability of the raw material ensures the human safety of the final target compound.

Specifically, the present invention uses an organic amine compound used for a cosmetic composition in an organic salt of terephthalylidene dicamphor sulfonic acid obtained as a neutralized product having a pH of 5.0 to 9.0, preferably a pH of 6.1 to 8.0, by combining the organic amine compound with terephthalylidene dicamphor sulfonic acid in an equivalent ratio of 1:2.

In the embodiments of the present invention, tromethamine, triethanolamine, or 2-Amino-2-methyl-1-propanol (AMP) is used as a preferred example, but it is not limited thereto.

As another organic amine compound, as an alkaline amino acid, any one selected from the group consisting of arginine, lysine, histidine, citrulline, and an amino acid derivative in which a carboxyl group of an amino acid is substituted and only a primary amine group remains may be used. As an example of the amino acid derivative in which a carboxyl group of an amino acid is substituted and only a primary amine group remains, N-acetyl-glutamine isostearyl (isostearyl acetyl glutaminate) may be used.

In the embodiments of the present invention, arginine, lysine and histidine are used for explanation, but it is not limited thereto.

In addition, as the organic amine compound of the present invention, a polyamine may be preferably used. More preferably, any one selected from the group consisting of polyethyleneimine (PEI), spermidine, spermine, cadaverine, and putrescine may be used.

Polyethyleneimine is the most well-known polyamine, has the highest cationic density among currently existing materials and particularly, it is a water-soluble polymer.

In addition, spermidine is found in various foods such as soybean, wheat, and meat, while spermine is a polyamine structurally similar to spermidine, performing various physiological functions within cells. Cadaverine is a polyamine found in rotten meat, from which its name is derived, and putrescine is another biologically derived polyamine found in various organisms, all of which are used in the present invention.

The above-described organic amine compound is combined in a stoichiometric ratio of two equivalents to terephthalylidene dicamphor sulfonic acid to form an organic salt of terephthalylidene dicamphor sulfonic acid.

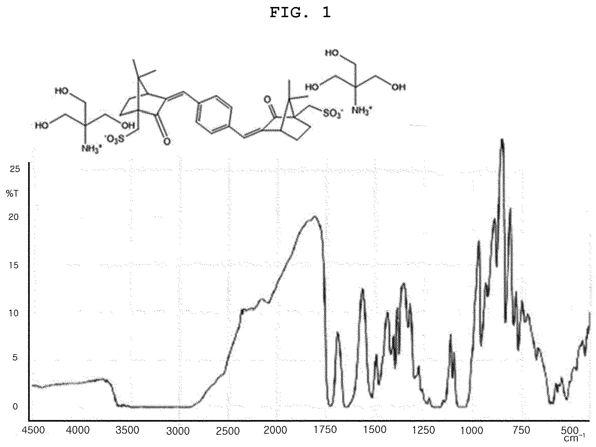

**FIG.** 1 shows infrared spectrum results for the terephthalylidene dicamphorsulfonic acid·2 tromethamine organic salt of the present invention, confirming synthesis of the organic salt.

The organic salt of terephthalylidene dicamphor sulfonic acid of the present invention may be provided in the form of an aqueous solution or powder according to the desired formulation during the manufacture of cosmetics and may be provided in a stable salt form, so that a separate neutralizing agent is not required during the manufacture of cosmetics, making it convenient and safe, and enabling consistent prescription of cosmetic formulations.

In addition, the organic salt of terephthalylidene dicamphor sulfonic acid obtained through the preparation method of the present invention provides long-term stability.

The present invention provides a preparation method an organic salt of terephthalylidene dicamphor sulfonic acid in a first embodiment, the preparation method an organic salt of terephthalylidene dicamphor sulfonic acid including: dissolving an alkaline organic amine in a purified aqueous solution of terephthalylidene dicamphor sulfonic acid at a temperature lower than or equal to 30°C, adjusting the pH of the solution, and then filtering the solution using a microfilter to obtain a clear aqueous solution.

At this time, the pH of the organic salt of terephthalylidene dicamphor sulfonic acid is finally adjusted to be obtained in the range of 5.0 to 9.0, most preferably in the range of 6.1 to 8.0.

An aqueous solution containing a stable formulation of the organic salt of terephthalylidene dicamphor sulfonic acid may be provided.

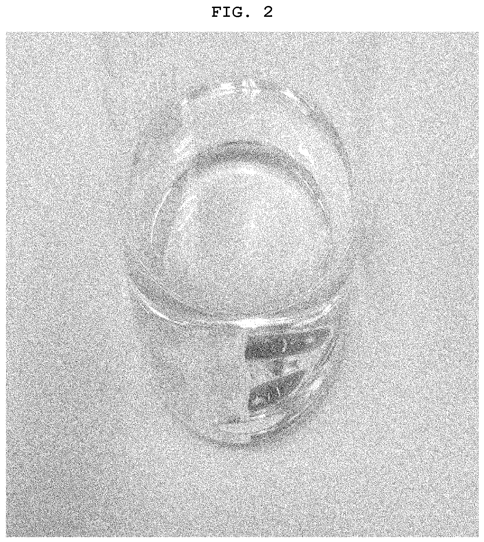

As an example, **FIG. 2** shows photograph of an aqueous solution of terephthalylidene dicamphorsulfonic acid·2 tromethamine of Example 1. The organic salt of terephthalylidene dicamphor sulfonic acid is more stable in the neutral solution of the organic salt of terephthalylidene dicamphor sulfonic acid than in the acidic solution, and especially, since it is a neutral solution, it does not spoil, and the stability thereof is improved when a preservative such as 1,2-hexanediol is used.

At this time, the aqueous solution may further comprise one or more preservatives selected from the group consisting of 1,2-alkanediol, ethylhexyl glycerin, hydroxyacetophenone, glycerin esters, parabens, and sodium benzoate. By further including the preservative, the formulation stability of the salt may be improved.

More specifically, for the organic salt of terephthalidene dicamphor sulfonic acid obtained, by using 1-2.5% of 1,2-hexanediol, 1-5% of 1,2-pentanediol, 0.1-1% of 1,2-octanediol, and 0.1-1% of ethylhexylglycerin, either individually or in combination, the storage stability of the organic salt aqueous solution of terephthalidene dicamphor sulfonic acid can be improved.

In the preparation method of a second embodiment of the present invention, a clear aqueous solution of the organic salt of the terephthalylidene dicamphor sulfonic acid obtained in the first embodiment is prepared, the aqueous solution is added dropwise to an organic solvent such as alcohol or acetone or a mixture thereof in an amount four times the volume of the aqueous solution to precipitate crystals, and the crystals are filtered and dried and vacuum-dried to obtain in a powder form.

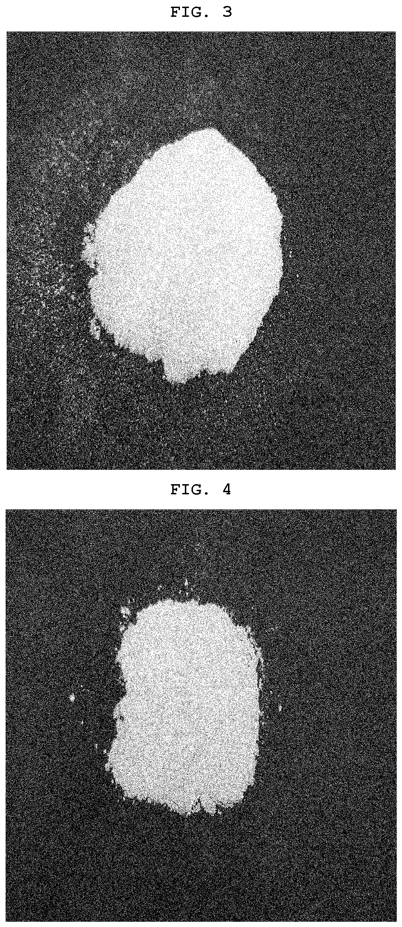

**FIG. 3** is a photograph of the crystals of terephthalylidene dicamphorsulfonic acid·2 tromethamine of the present invention, and 100 g of the clear aqueous solution of the organic salt of terephthalylidene dicamphor sulfonic acid obtained in the first embodiment was slowly added dropwise to a four-necked round bottom flask containing 100 to 500 ml of alcohol or acetone or a mixed solvent thereof while stirring to precipitate a salt, and the precipitated crystals were filtered, washed with acetone, and vacuum-dried to obtain an organic salt of tromethamine of terephthalylidene dicamphor sulfonic acid.

In addition, in the preparation method of a third embodiment of the present invention, a clear aqueous solution of the organic salt of terephthalylidene dicamphor sulfonic acid obtained in the first embodiment is prepared, and the aqueous solution is freeze-dried to obtain in a powder form.

**FIG. 4** shows the powder form of terephthalylidene dicamphorsulfonic acid·2 tromethamine of the present invention. As described above, the present invention provides an aqueous solution obtained from the preparation method of an organic salt of terephthalylidene dicamphor sulfonic acid to be used as it is or in various forms including the crystal form, by precipitating the aqueous solution in an organic solvent, or the powder form, by freeze-drying it, so that it may be selectively used according to the desired formulation when producing cosmetics.

In particular, the crystal form of the organic salt of terephthalylidene dicamphor sulfonic acid is present as a solid, and therefore it is more stable than an acidic liquid and a neutral solution, and thus can be stored for a long time even at a high temperature such as 45°C.

Through the preparation method of the present invention, problems such as product stability problems and corrosion of cosmetics production equipment due to the use of a strongly acidic substance with a pH of 1 or lower, which is the disadvantage of the conventionally used terephthalylidene dicamphor sulfonic acid, contamination of cosmetics with heavy metals caused thereby, and difficulty in consistent product implementation that may occur during the cosmetics formulation process can be improved, and the safety to the user's body and during the work required for cosmetics production is provided.

Hereinafter, the present invention will be described in more detail through examples.

These examples are intended to explain the present invention in more detail, and the scope of the present invention is not limited to these examples.

### <Example 1>

According to the analytical method of the US Pharmacopoeia, 1000 g (0.597 mol) of an aqueous solution of terephthalylidene dicamphor sulfonic acid (Ecamsule) with the purity of 33.6% was put into a 2 L four-necked round bottom flask, 144.64 g (1.194 mol) of tromethamine having a molecular weight of 121.14 g/mol was added while stirring to completely dissolve the solute, and the pH of the resulting solution was adjusted to 6.6. The solution was filtered through a filter paper to obtain a clear aqueous solution containing terephthalylidene dicamphorsulfonic acid·2 tromethamine represented by Chemical Formula 1-1 below.

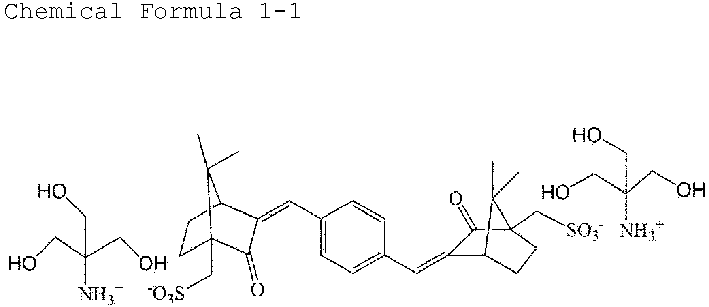

### <Example 2>

200 g of the aqueous solution of terephthalylidene dicamphorsulfonic acid·2 tromethamine obtained in Example 1 was slowly added dropwise to a beaker containing 800 ml of acetone prepared in advance at room temperature for 30 minutes to precipitate crystals. Stirring was continued for additional two hours until the crystals were completely precipitated, and the crystals were filtered and washed to obtain the target product, the crystals of terephthalylidene dicamphorsulfonic acid·2 tromethamine. The resulting product was vacuum-dried to obtain 79.79 g of the target product as a solid (yield 95%).

### <Example 3>

200 g of the aqueous solution of terephthalylidene dicamphorsulfonic acid-2 tromethamine obtained in Example 1 was placed in a freeze dryer (Lyovapor^{™} L-300 model) and freeze-dried at ±1°C for 15 hours to obtain 83.98g of terephthalylidene dicamphorsulfonic acid·2 tromethamine as a solid in the form of white powder.

### <Example 4>

According to the analytical method of the US Pharmacopoeia, 1000 g (0.597 mol) of an aqueous solution of terephthalylidene dicamphor sulfonic acid with the purity of 33.6% was put into a 2 L four-necked round bottom flask, 178.13 g (1.194 mol) of triethanolamine having a molecular weight of 149.19 g/mol was added while stirring to completely dissolve the solute, and the pH of the resulting solution was adjusted to 6.6. The solution was filtered through a filter paper to obtain a clear aqueous solution containing terephthalylidene dicamphorsulfonic acid·2 triethanolamine.

### <Example 5>

200 g of the aqueous solution of terephthalylidene dicamphorsulfonic acid·2 triethanolamine obtained in Example 4 was slowly added dropwise to a beaker containing 800 ml of acetone prepared in advance at room temperature for 30 minutes to precipitate crystals. Stirring was continued for additional two hours until the crystals were completely precipitated, and the crystals were filtered and washed to obtain the target product, the crystals of terephthalylidene dicamphorsulfonic acid·2 triethanolamine. The crystals of terephthalylidene dicamphorsulfonic acid·2 triethanolamine were vacuum-dried to obtain 83.79 g of the target product as a solid (yield 96%).

### <Example 6>

200 g of the aqueous solution of terephthalylidene dicamphorsulfonic acid·2 triethanolamine obtained in Example 4 was placed in a freeze dryer (Lyovapor^{™} L-300 model) and freeze-dried at ±1°C for 15 hours to obtain 87.19g of terephthalylidene dicamphorsulfonic acid·2 triethanolamine as a solid in the form of white powder.

### <Example 7>

According to the analytical method of the US Pharmacopoeia, 1000 g (0.597 mol) of an aqueous solution of terephthalylidene dicamphor sulfonic acid with the purity of 33.6% was put into a 2 L four-necked round bottom flask, 208 g (1.194 mol) of arginine having a molecular weight of 174.21 g/mol was added while stirring to completely dissolve the solute, and the pH of the resulting solution was adjusted to 6.6. The solution was filtered through a filter paper to obtain a clear aqueous solution of terephthalylidene dicamphorsulfonic acid·2 arginine.

### <Example 8>

200 g of the aqueous solution of terephthalylidene dicamphorsulfonic acid·2 arginine obtained in Example 7 was slowly added dropwise to a beaker containing 800 ml of acetone prepared in advance at room temperature for 30 minutes to precipitate crystals. Stirring was continued for additional two hours until the crystals were completely precipitated, and the crystals were filtered and washed to obtain the target product, the crystals of terephthalylidene dicamphorsulfonic acid·2 arginine. The resulting product was vacuum-dried to obtain 85.57 g of the target product as a solid (yield 95%).

### <Example 9>

200 g of the aqueous solution of terephthalylidene dicamphorsulfonic acid·2 arginine obtained in Example 7 was placed in a freeze dryer (Lyovapor^{™} L-300 model) and freeze-dried at ±1°C for 15 hours to obtain 90.07 g of terephthalylidene dicamphorsulfonic acid·2 arginine as a solid in the form of white powder.

### <Example 10>

According to the analytical method of the US Pharmacopoeia, 1000 g (0.597 mol) of an aqueous solution of terephthalylidene dicamphor sulfonic acid with the purity of 33.6% was put into a 2 L four-necked round bottom flask, 106.3 g (1.194 mol) of 2-amino-2-methyl-1-propanol (AMP) having a molecular weight of 89.136 g/mol was added while stirring to completely dissolve the solute, and the pH of the resulting solution was adjusted to 6.6. The solution was filtered through a filter paper to obtain a clear aqueous solution of terephthalylidene dicamphorsulfonic acid·2AMP.

### <Example 11>

200 g of the aqueous solution of terephthalylidene dicamphorsulfonic acid·2AMP obtained in Example 10 was slowly added dropwise to a beaker containing 800 ml of acetone prepared in advance at room temperature for 30 minutes to precipitate crystals. Stirring was continued for additional two hours until the crystals were completely precipitated, and the crystals were filtered and washed to obtain the target product, the crystals of terephthalylidene dicamphorsulfonic acid·2AMP. The resulting product was vacuum-dried to obtain 75.97 g of the target product as a solid (yield 95%).

### <Comparative Example 1>

According to the analytical method of the US Pharmacopoeia, 1000 g (0.597 mol) of an aqueous solution of terephthalylidene dicamphor sulfonic acid with the purity of 33.6% was put into a 2 L four-necked round bottom flask, and sodium hydroxide was added while stirring to precipitate a white solid. The terephthalylidene dicamphor sulfonic acid· 2 Na salt obtained using a NaOH inorganic salt as a neutralizing agent had a low solubility in water of 2% to 3% at room temperature, and therefore it was difficult to prepare a high-concentration aqueous solution of 30% or more, and at the same time, the UV absorption effect was also reduced, making it unsuitable for use as a UV-blocker.

### <Experimental Example 1> Evaluation of sustained stability of terephthalylidene dicamphor sulfonic acid salt

The aqueous solution of terephthalylidene dicamphor sulfonic acid-2 tromethamine of Example 1, an aqueous solution containing the aqueous solution of Example 1 mixed with 2.5% 1,2-hexanediol, and the crystals of terephthalylidene dicamphorsulfonic acid·2 tromethamine of Example 2 were used, and after converting the 33.6% terephthalylidene dicamphor sulfonic acid (TDSA) aqueous solution to the same equivalent, the content was analyzed by HPLC every 3 months for up to 12 months and every 6 months thereafter for up to 24 months to conduct a stability evaluation. The purity test was performed according to the US Pharmacopoeia 44 Ecamsule content test method. At this time, the stability test was performed in a constant temperature room maintained at 45°C under dark conditions blocked from light.

Regarding the HPLC sample preparation method, in the case of the 33.6% aqueous solution of terephthalylidene dicamphor sulfonic acid, 300 mg of the 33.6% aqueous solution of terephthalylidene dicamphor sulfonic acid was put into a 100-mL graduated flask, the volume was adjusted to 100 mL with purified water, 5 mL of the resulting solution was again taken and put into a 100-mL graduated flask, and the volume was adjusted to 100 mL with purified water to prepare the sample.

Also, in the case of the terephthalylidene dicamphor sulfonic acid·2 tromethamine aqueous solution of Example 1 and the aqueous solution of Example 1 containing 2.5% of 1,2-hexanediol, samples were prepared in the same manner as above to conduct a content test.

100 mg of the crystals of terephthalylidene dicamphorsulfonic acid·2 tromethamine of Example 2 were taken and put into a 100-mL graduated flask, the volume was adjusted to 100 mL with purified water, 5 mL of the resulting solution was again taken and put into a 100-mL graduated flask, and the volume was adjusted to 100 mL with purified water to prepare the sample. The solution was subjected to measurement using Shimadzu HPLC, and the results are shown in **Table 1** below.

**[Table 1]**

| Results of stability evaluation | | | | | | | |
|---|---|---|---|---|---|---|---|
| | Content measured by HPLC (%) | | | | | | |
| Classification | Month 0 | Month 3 | Month 6 | Month 9 | Month 12 | Month 18 | Month 24 |
| 33.6% aqueous solution of TDSA | 33.6 | 33.3 | 33.0 | 32.5 | 32.1 | 31.4 | 30.5 |
| Aqueous solution of Example 1 | 29.28 | 29.25 | 29.19 | 29.14 | 28.97 | 28.71 | 28.32 |
| Aqueous solution of Example 1 containing 1,2-hexanediol | 28.62 | 28.60 | 28.57 | 28.50 | 28.43 | 28.36 | 28.29 |
| Solid of Example 2 | 69.80 | 69.80 | 69.60 | 69.80 | 69.80 | 69.70 | 69.70 |

From the results shown in Table 1, it was confirmed that the aqueous solution of terephthalylidene dicamphor sulfonic acid was a strong acidic solution and that its purity was reduced when stored for a long time at a high temperature of 45°C.

On the other hand, it was confirmed that the aqueous solution of the neutral salt of terephthalylidene dicamphorsulfonic acid·2 tromethamine of Example 1 was more stable than the acidic aqueous solution, and furthermore, it was even more stable when it was included in the aqueous solution containing 1,2-hexanediol. In addition, it was confirmed that the solid obtained by crystallizing the salt of terephthalylidene dicamphorsulfonic acid·2 tromethamine was stable with almost no decrease in the content not only at a high temperature of 45°C but also under long-term storage conditions of 24 months.

Although the present invention has been described in detail only with respect to the described specific examples, it is obvious to those skilled in the art that various modifications and corrections are possible within the scope of the technical idea of the present invention, and it is obvious that such modifications and corrections fall within the scope of the appended claims.

## Claims

1. An organic salt of terephthalylidene dicamphor sulfonic acid represented by Chemical Formula 1 below, which is obtained as a neutralized product by stoichiometrically combining an organic amine compound to both terminals of terephthalylidene dicamphor sulfonic acid, useful as a UV-blocking cosmetic compositions: 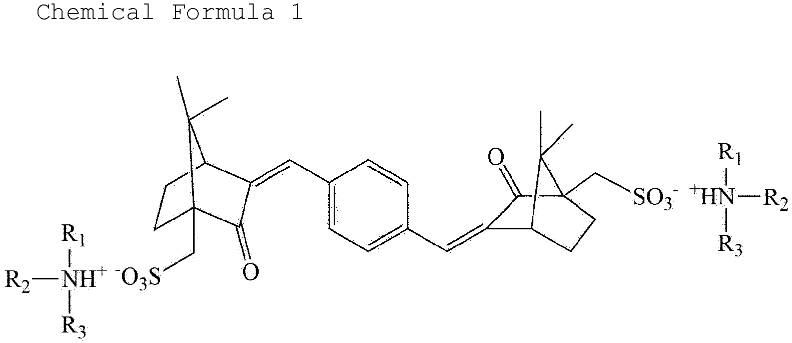 wherein, R₁, R₂, and R₃ are the same or different from each other and independently selected from one or more substituents consisting of hydrogen, hydroxy, amino, carboxy, C₁-C₁₀ alkyl, C₁-C₁₀ hydroxyalkyl, C₁-C₁₀ heteroalkyl, heterocycloalkyl having 20 or fewer carbon atoms, aryl having 20 or fewer carbon atoms, C₁-C₁₀ alkyl containing heterocycloalkyl having 20 or fewer carbon atoms, and C₁-C₁₀ alkyl containing aryl having 20 or fewer carbon atoms.

2. The organic salt of terephthalylidene dicamphor sulfonic acid according to claim 1, wherein the organic amine compound is any one selected from the group consisting of tromethamine, triethanolamine, and 2-amino-2-methyl-1-propanol (AMP).

3. The organic salt of terephthalylidene dicamphor sulfonic acid according to claim 1, wherein the organic amine compound is any one alkaline amino acid selected from the group consisting of arginine, lysine, histidine, citrulline, and an amino acid derivative in which a carboxyl group of an amino acid is substituted and only a primary amine group remains.

4. The organic salt of terephthalylidene dicamphor sulfonic acid according to claim 1, wherein the organic amine compound is any one polyamine selected from the group consisting of polyethyleneimine, spermidine, spermine, cadaverine, and putrescine.

5. The organic salt of terephthalylidene dicamphor sulfonic acid according to claim 1, wherein the organic salt of terephthalylidene dicamphor sulfonic acid is in the form of an aqueous solution or powder.

6. A method for preparing an organic salt of terephthalylidene dicamphor sulfonic acid represented by Chemical Formula 1 below, which is obtained as a clear aqueous solution by dissolving an organic amine compound in a purified terephthalylidene dicamphor sulfonic acid aqueous solution, adjusting the pH of the solution, and then filtering: 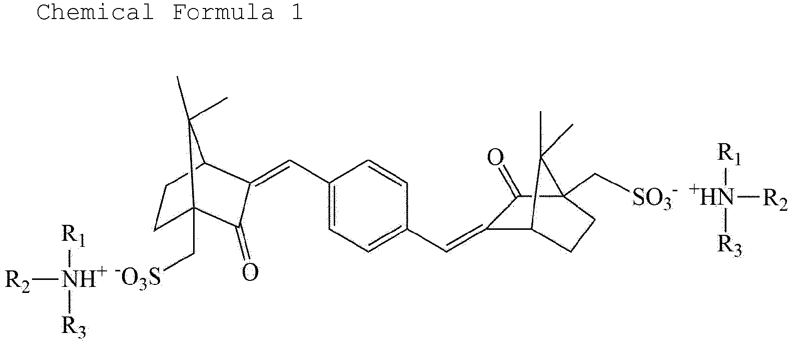 wherein, R₁, R₂, and R₃ are as defined in claim 1.

7. The method according to claim 6, further comprising adding the obtained clear aqueous solution dropwise to an organic solvent to precipitate crystals, and vacuum drying the crystals to obtain a powder form.

8. The method according to claim 6, further comprising freeze-drying the obtained clear aqueous solution to obtain a powder form.

9. The method according to claim 6, wherein the aqueous solution further comprises one or more preservatives selected from the group consisting of 1,2-alkanediol, ethylhexyl glycerin, hydroxyacetophenone, glycerin esters, parabens, and sodium benzoate.
